# EUROPEAN PATENT SPECIFICATION

(11) **EP 3 434 170 B1**
(45) Date of publication and mention of the grant of the patent: **17.03.2021**
(21) Application number: 16895438.6
(22) Date of filing: 25.03.2016
(51) Int. Cl.: A61B 1/00, A61B 34/30, A61B 90/00, A61B 1/05, A61B 1/313

(54) **ENDOSCOPE APPARATUS AND ENDOSCOPE SYSTEM INCLUDING THE SAME**
ENDOSKOPVORRICHTUNG UND ENDOSKOPSYSTEM MIT DEMSELBEN
ENDOSCOPE ET SYSTÈME D'ENDOSCOPE COMPRENANT LE MÊME

(43) Date of publication of application: 30.01.2019
(73) Proprietor: Nikon Corporation, Tokyo 108-6290 (JP); Medicaroid Corporation, Kobe-shi, Hyogo 650-0047 (JP)
(72) Inventor: ISHIKAWA Tetsuro, Tokyo 108-6290 (JP); HAMATANI Masato, Tokyo 108-6290 (JP); NAGATSUKA Jun, Tokyo 108-6290 (JP); WATANABE Shunji, Tokyo 108-6290 (JP); INOUE Jiro, Tokyo 108-6290 (JP); HASHIMOTO Yasuhiko, Kobe-shi Hyogo 650-0047 (JP); TANAKA Hirofumi, Kobe-shi Hyogo 650-0047 (JP); TAMURA Yoshiyuki, Kobe-shi Hyogo 650-0047 (JP); HIRATSUKA Mitsuichi, Kobe-shi Hyogo 650-0047 (JP)
(74) Representative: Hoffmann Eitle
(86) International application number: PCT/JP2016/059672
(87) International publication number: WO 2017/163407

(56) References cited:
- WO-A1-2010/050243
- JP-A- H1 043 127
- JP-A- H07 327 921
- JP-A- H08 332 169
- JP-A- H11 113 836
- JP-A- 2004 289 225
- JP-A- 2006 288 869
- JP-A- 2008 528 130
- US-A1- 2014 179 997
- US-A1- 2015 045 619

## Description

### Technical Field

This disclosure relates to an endoscope apparatus and a surgical system that includes the endoscope apparatus.

### Background Art

Recently, Minimally Invasive Surgery (MIS) technique has attracted attention. Minimally Invasive Surgery technique is a technique for performing surgery on a patient by using a camera and an elongated surgical instrument introduced into a surgical site inside a body through a small incision site via a trocar sleeve or a cannula. The surgical site often includes a body cavity such as an abdomen of the patient. The body cavity is expanded as necessary using a transparent fluid such as an insufflation gas. Typically, in Minimally Invasive Surgery, an operator such as a doctor uses an end effector of an elongated surgical instrument to operate a handle of the surgical instrument, and operates tissues while observing the surgical site on a video monitor.

A common form of Minimally Invasive Surgery is an endoscopy. A laparoscopy is a kind of the endoscopy to execute a minimally invasive examination and a surgery inside an abdominal cavity. In a typical laparoscopic surgery, a cannula sleeve is passed through a small (generally, equal to or less than 1.27 cm (1/2 inches)) incision site to dispose an inlet port for a laparoscopic surgical instrument. A gas is insufflated into an abdomen of a patient to form a space having a certain volume inside the abdominal cavity.

The laparoscopic surgical instrument includes a laparoscope (a kind of an endoscope applied for observing a surgical field inside the abdominal cavity) and operation tools. The operation tools are similar to those used in conventional incision surgery excluding that an operation end or an end effector of each tool is separated from a handle of the tool by a tool shaft.

Relating to such surgical system having the laparoscope, for example, Patent Literature 1 discloses a minimally invasive robotic surgical system where a robot manipulator like a manipulator for moving surgical instruments are used to hold the laparoscopes to align them with a desired surgical site in a patient body.

### Citation List

### Patent Literature

Patent Literature 1: JP 2014-028296 A
US 2014/179997 A1 discloses a surgical robot system with at least two robot arms, on each of which is arranged at least one endoscope for a minimally invasive surgery, wherein the first endoscope on the first robot arm comprises a main support means and which comprises at the distal end at least one lighting unit and two image-taking devices, and a trocar, and wherein the second endoscope on to the second robot arm comprises a main support means, a trocar, and an auxiliary support means.
US 2015/045619 A1 discloses a system for mosaicing endoscope images having an endoscope, a computer and a monitor. The endoscope has a relatively wide viewing angle such as 30 degrees to capture video images of a target area. The computer determines feature points on the captured video frames, aligns the video images in a common reference frame and displays them as one mosaiced image while dynamically extending the scene synchronous with the movement of the endoscope. The system creates a distortion free near real-time panorama of the endoscope scene using the wide angle view endoscope, and is useful for physicians while performing endoscopic procedures since the field of view can be extended to provide a better visual-spatial orientation.

### Summary of Invention

In the conventional surgical system as disclosed in Patent Literature 1, a range of images obtained on the surgical site and its peripheral site is limited. For example, it is difficult to obtain an image behind (for example, a part (near a cannula) close to a base of a tubular housing of the laparoscope) the camera disposed on the laparoscope.

In order to solve the problem of the present invention an endoscope apparatus is defined according to the features of independent claim 1 and the dependent claims. Further, an endoscope system is provided according to the features of claim 11.
(i) An endoscope apparatus according to an aspect of the disclosure includes three or more endoscopes and a controller. The three or more endoscopes each include an imaging device on a distal end portion of a tubular housing (not limited to a cylindrical shape). The three or more endoscopes each obtain an image in a body of a subject. The controller is electrically coupled to the three or more endoscopes. The controller processes the images obtained by the respective three or more endoscopes. The controller uses position information on the respective imaging devices of the three or more endoscopes and feature points of the respective images obtained by the respective imaging devices to join the respective images together, to generate a composite image.
(ii) An endoscope apparatus of another aspect of the disclosure includes an endoscope, a rotation driver, and a controller. The endoscope includes an imaging device on a distal end portion of a tubular housing. The endoscope obtains an image in an abdominal cavity of a subject. The rotation driver is configured to rotate at least the distal end portion of the tubular housing of the endoscope. The controller is electrically coupled to the endoscope. The controller processes the image obtained by the endoscope. The imaging device has an optical axis having a predetermined angle with a rotation axis of the distal end portion of the tubular housing. The controller joins the images obtained by the imaging device while rotating the distal end portion of the tubular housing of the endoscope together, to generate a composite image.
(iii) An endoscope system according to an aspect of the disclosure includes a robot cart, a console apparatus, and a display apparatus. The robot cart includes an endoscope apparatus and a surgical arm to which a surgical instrument is mounted. The console apparatus transmits an instruction to manipulate an endoscope and the surgical arm of the robot cart. The display apparatus displays an image taken by the endoscope apparatus on a display screen. The endoscope apparatus includes three or more endoscopes and a controller. The three or more endoscopes each include an imaging device on a distal end portion of a tubular housing. The three or more endoscopes each obtain an image in a body of a subject. The controller is electrically coupled to the three or more endoscopes. The controller processes the images obtained by the respective three or more endoscopes. The controller uses feature points of the respective images obtained by the respective imaging devices to join the respective images together, to generate a composite image.
(iv) An endoscope system of another aspect of the disclosure includes a robot cart, a console apparatus, and a display apparatus. The robot cart includes an endoscope apparatus and a surgical arm to which a surgical instrument is mounted. The console apparatus transmits an instruction to manipulate an endoscope and the surgical arm of the robot cart. The display apparatus displays an image taken by the endoscope apparatus on a display screen. The endoscope apparatus includes an endoscope, a rotation driver, and a controller. The endoscope includes an imaging device on a distal end portion of a tubular housing. The endoscope obtains an image in a body of a subject. The rotation driver is configured to rotate at least the distal end portion of the tubular housing of the endoscope. The controller is electrically coupled to the endoscope. The controller processes the image obtained by the endoscope. The imaging device has an optical axis having a predetermined angle with a rotation axis of the distal end portion of the tubular housing. The controller joins the images obtained by the imaging device while rotating the distal end portion of the tubular housing of the endoscope together, to generate a composite image.
(v) Further features related to this disclosure are clarified from the explanations of this description and the accompanying drawings. The aspects of this disclosure can be achieved and realized by components and combinations of various components, and the following detail description and aspects of accompanying claims. It should be understood that the explanations of this description are merely typical examples and therefore do not limit the claims and application examples of the present invention by any means.

### Brief Description of Drawings

Fig. 1 is a drawing illustrating an exemplary schematic configuration of a surgical system (also referred to as a surgical robot system) 1 according to the embodiment.
Fig. 2 is a drawing illustrating an exemplary internal configuration of a patient-side cart 20 according to a first embodiment.
Fig. 3 is a flowchart describing a composite image generation process in the first embodiment.
Fig. 4 are drawings schematically illustrating visual fields of imaging devices to describe a reason why three or more endoscopes are disposed in this disclosure.
Fig. 5 is a drawing illustrating an exemplary configuration of a patient-side cart 20 according to a second embodiment.
Fig. 6 is a drawing illustrating a distal end portion of an endoscope arm 27 on which a transparent sheath 61 having a rounded distal end portion is covered.
Fig. 7 is a drawing describing a visual field ensured in rotating the distal end portion of the endoscope arm 27.
Fig. 8 is a flowchart describing a composite image generation process in the second embodiment.
Fig. 9 is a drawing illustrating an exemplary configuration of a patient-side cart 20 according to a third embodiment.

### Description of Embodiment of the disclosure

The following describes embodiments of this disclosure with reference to the accompanying drawings. The protected subject-matter is defined in claims 1 - 11. The accompanying drawings represent functionally identical elements by identical reference numerals in some cases. Although the accompanying drawings illustrate specific embodiments and examples of mounting according to a principle of this disclosure, these drawings are for understanding of this disclosure and never used for limited interpretation of this disclosure.

While the embodiments give the explanation in detail enough for a person skilled in the art to carry out this disclosure, it is necessary to understand that other mountings and forms are possible and that changes in configurations and structures and substitutions of various components can be made without departing from the scope of the technical idea of this disclosure. Therefore, the following description should not be interpreted to be limited.

Further, as described later, the embodiments of this disclosure may be mounted by software running on a general-purpose computer, by dedicated hardware, or by a combination of software and hardware.

The following describes each process in the embodiments of this disclosure having "each processor (for example, an image processor) as a program" as a subject (operation subject). However, the description may be given with a processor as a subject because the program is executed by the processor (CPU and the like: simply, can be referred to as a controller) to perform specified processes using a memory and a communication port (a communication controller).

### (1) First Embodiment

A first embodiment discloses a surgical system that includes, for example, a patient-side cart (also referred to as a surgical robot) that includes an endoscope apparatus including three or more endoscope arms, and a console apparatus for manipulating the patient-side cart. For example, the endoscope apparatus uses feature points of respective images obtained by respective imaging devices of the three or more endoscopes to generate a composite image by joining the respective images together, and indicates the composite image on a display screen. The composite image generated from the images in three visual fields and provided to the operator ensures providing the image in a wide range, thus allowing the operator to confirm various sites by a visual check during surgery (ensuring an endoscopic surgery while having a large visual field as in a laparotomy). The three or more endoscopes arranged on appropriate positions ensures obtaining images on approach routes of the endoscopes in real-time when the endoscopes are inserted into a body (into an abdominal cavity or into a thoracic cavity). In this case, it can be confirmed whether the endoscopes squeeze peripheral organs in the body or not.

As a lens of each imaging device included in the three or more endoscopes, for example, a lens having a viewing angle equal to or more than 90 degrees (for example, a foveal lens) can be used. In the case where the foveal lens is used, the visual field of approximately 180 degrees is ensured, and the center of the visual field is clearly visible while a peripheral region of the visual field is vaguely visible as a visual field of human.

While the approach route of the endoscope can be confirmed in real-time as described above, it is not always possible to insert the three or more endoscopes from optimal positions. Therefore, for example, the images before composition obtained by the respective imaging devices since the three or more endoscopes were inserted into a body of a patient (a subject) and the composite images since the three or more endoscopes were inserted into the body of the subject are stored in a memory, for example. Then, in response to an instruction input by the operator, the composite images and the images before composition at a predetermined time point can be indicated on the display screen from the memory. For example, indicating past images of the approach route of the endoscope together with the images during surgery allows the operator to confirm whether a mistake has not been made in the insertion process of the endoscope.

### <Configuration of Surgical System>

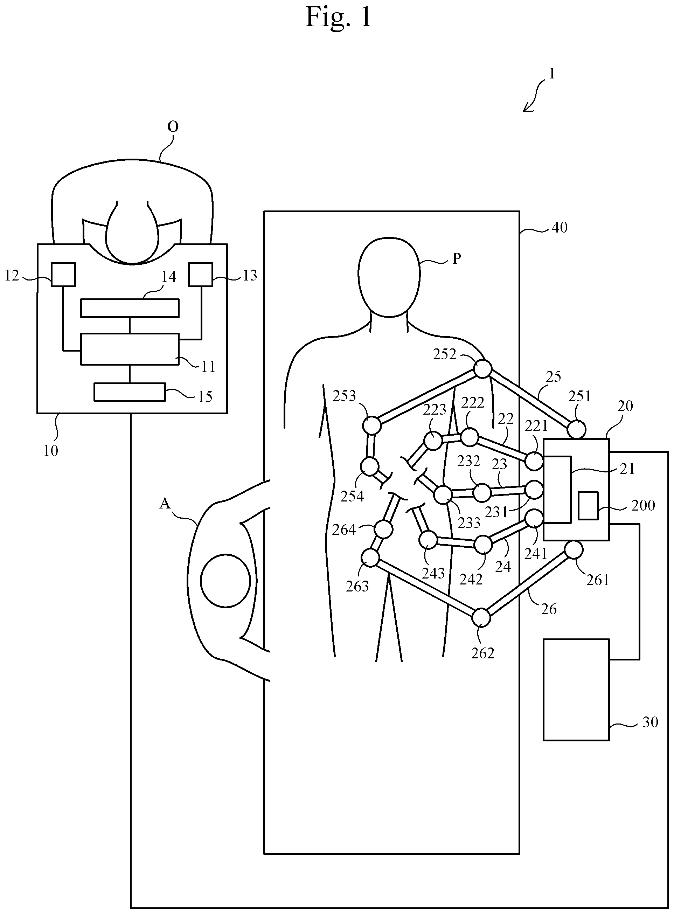

Fig. 1 is a drawing illustrating an exemplary schematic configuration of a surgical system (also referred to as a surgical robot system) 1 according to the embodiment.

The surgical system 1 includes, for example, a console apparatus 10 manipulated by an operator (for example, a surgeon) O, a patient-side cart (also referred to as a surgical robot cart) 20 for performing a predetermined surgery on a patient (also referred to as a subject) P lying on a surgical table 40 based on instructions from the console apparatus 10, and a display apparatus 30. In Fig. 1, the console apparatus 10, the patient-side cart 20, and the display apparatus 30 are coupled by, for example, wire, while a configuration to couple them by a wireless network (Internet, a wireless LAN, and the like) may be employed.

The console apparatus 10 includes a processor (also referred to as a CPU or a controller) 11 that controls predetermined operations and processes, right and left manipulators 12 and 13 with which the operator O remotely manipulates the endoscope arm and a surgical arm mounted on the patient-side cart 20, a display 14 that indicates images from the endoscope described below, and a communication apparatus 15 that transmits and receives information and data to/from the patient-side cart 20. The console apparatus 10 may include at least one foot pedal (not illustrated). The right manipulator 12 and the left manipulator 13 are, for example, grip input mechanisms that the operator O grips with his/her own hands to manipulate the patient-side cart 20. The operator O can remotely manipulate one or a plurality of surgical arms or endoscope arms mounted on the patient-side cart 20 by manipulating input apparatuses of the right manipulator 12 (the grip input mechanism) and the left manipulator 13 (the grip input mechanism) of the console apparatus 10. This ensures desired operations of the surgical instruments (including end effectors of the surgical instruments) mounted on the surgical arms and the endoscopes mounted on the endoscope arms. Accordingly, the console apparatus 10 functions as a master controller to manipulate the patient-side cart 20 when a desired surgical treatment is executed. In some cases, commands for controlling functions other than the operations of the surgical instruments and the endoscopes mounted on the patient-side cart 20 are provided to the patient-side cart 20 via the console apparatus 10. For example, the foot pedal (not illustrated) can be used to transmit a cautery command for supplying electrosurgical energy to electrosurgical instruments mounted on the surgical arms of the patient-side cart 20 from the console apparatus 10 to the patient-side cart 20. However, the surgical system 1 is not only configured to manipulate the endoscope and the surgical instrument mounted on the patient-side cart 20 via the console apparatus 10, but also may be configured to achieve manipulations of the surgical instruments and the like using, for example, a cart-side manipulator 200 of the patient-side cart 20. For example, in some cases, the endoscope and the surgical instrument of the patient-side cart 20 are manipulated by a surgical assistant A or another operator (for example, a surgeon) who directly manipulates the patient-side cart 20. The input apparatus of the console apparatus 10 may employ an aspect of, for example, a joystick, a motion sensor, a switch, or a thumb/finger control, other than a gripping mechanism or a foot pedal. The above-described "end effector" means an actually operating part (usually, a distal end) of the surgical instrument, and may include, for example, forceps, a grasper, scissors, an anastomosis instrument, an imaging lens, and a needle holder. The end effector for the endoscope (a laparoscope or a thoracoscope) includes, for example, a lens that can be optically coupled to a camera and a lamp via a tool shaft, and light source (in a configuration described below, an illumination light source 212 is included in a main body of an endoscope apparatus 21), in some cases. For executing surgical procedures, the operator O or the surgical assistant A as an operating surgeon passes these operation tools or instruments to a surgical site inside the body via a cannula sleeve, and manipulates them from the outside of the abdomen.

The patient-side cart 20 includes, for example, the endoscope apparatus 21 that includes at least three endoscope arms 22 to 24, at least two surgical arms 25 and 26, and the cart-side manipulator 200. The endoscope arms 22 to 24 and the surgical arms 25 and 26 may be collectively referred to as a patient-side manipulator arm. The at least three endoscope arms 22 to 24 each have a distal end on which an imaging device (for example, a CMOS sensor or a CCD, and can be simply referred to as a camera, the same applies to below) is mounted. The imaging devices may be configured to be removable from the respective endoscope arms 22 to 24. On the at least two surgical arms 25 and 26, the surgical instruments are removably mounted corresponding to a surgical technique. The endoscope arms 22, 23, 24 and the surgical arms 25 and 26 include, for example, a plurality of joints 221 to 223, 231 to 233, 241 to 243, 251 to 254, and 261 to 264, respectively. The number of joints to be configured is not limited to the illustrated one, and may be conveniently configured. The joints each include a position detector that detects a rotation direction and rotation angle of each movable component of the arm, and an actuator for driving each movable component (not illustrated), and the position detector and the actuator are mutually associated. The position detector is, for example, an encoder, while a resolver or a potentiometer may be used. Based on information on the rotation direction and the rotation angle of each joint, information on a length between the joints (a length dimension of the movable component, predetermined), a length from the last joint on the arm distal end side to the arm distal end (predetermined), and lengths of the surgical instrument and the imaging device to be mounted (predetermined), a position of the imaging device of the endoscope in the body (for example, inside the abdominal cavity and inside the thoracic cavity) of the patient P, and positions of the distal ends of the surgical instruments and predetermined parts in the body of the patient P can be specified. Here, the endoscope is a concept including a laparoscope used for an abdominal surgery and a thoracoscope used for a pulmonary surgery, and can be referred to as, for example, a rigid endoscope.

The display apparatus 30 is installed to, for example, an open place independent of the patient-side cart 20 (not disposed as a display dedicated for the operator O like the display 14 of the console apparatus 10), and indicates the images obtained by the imaging device (for example, the CMOS sensor or the CCD) mounted on the endoscope arms 22 to 24 of the patient-side cart 20 on the display screen. This allows not only the operator O who remotely performs the surgery but also the surgical assistant A and other staff members to confirm a state during surgery. The behavior of the display apparatus 30 may be controlled by, for example, the CPU (also referred to as a processor or a controller) of the patient-side cart 20. In Fig. 1, the display apparatus 30 may be integrally disposed with the patient-side cart 20.

### <Configuration of Patient-Side Cart>

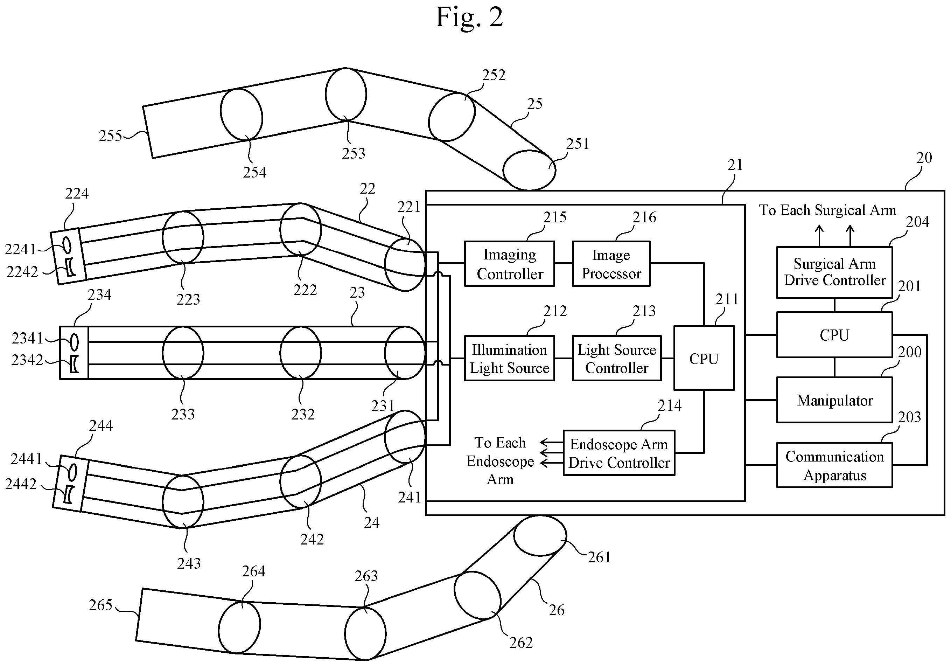

Fig. 2 is a drawing illustrating an exemplary internal configuration of the patient-side cart 20 according to the first embodiment. The patient-side cart 20 is a robot cart including a robot arm, and can be described dividing into the endoscope apparatus 21 that controls the endoscope and the endoscope arm and the other parts that control the surgical arm. A system where a robot cart that includes the endoscope apparatus 21 while not including the surgical arm is remotely manipulated by the console apparatus 10 is an endoscope system, and a system where the robot cart includes the surgical arm as well and the manipulation on the console apparatus 10 manipulates the surgical instrument and the endoscope to perform the surgery is a surgical system.

The endoscope apparatus 21 includes, for example, a CPU (also referred to as a processor or a controller) 211 that controls the entire apparatus, the illumination light source 212 that provides a light source to the imaging device of the endoscope, a light source controller 213 that controls a behavior of the illumination light source 212 responding to an instruction of the CPU 211, an imaging controller 215 that controls an imaging behavior of the imaging device of the endoscope, an image processor 216 that processes the images obtained by the imaging device, and an endoscope arm drive controller 214 that drives and controls each of the endoscope arms 22 to 24. On the endoscope apparatus 21 in the first embodiment, for example, at least three endoscope arms 22 to 24 are mounted. The endoscope apparatus 21 may be configured including the endoscope arms 22 to 24. While Fig. 2 is illustrated as a function block diagram of the patient-side cart 20, the light source controller 213, the endoscope arm drive controller 214, the imaging controller 215, the image processor 216, and a surgical arm drive controller 204 may be implemented as programs. In this case, a CPU 201 and the CPU 211 execute various programs to achieve a predetermined processing behavior.

The endoscope arms 22 to 24 include, for example, a plurality of joints 221 to 223, 231 to 233, and 241 to 243, optical adapters 224 to 244, and movable components (no reference numerals) between the respective joints. The plurality of joints 221 to 223, 231 to 233, and 241 to 243 include a plurality of position detectors (not illustrated) (for example, encoders) associated with the respective joints, and the position detectors can each detect a direction and an angle of rotation of each joint. The information on the rotation directions and angles of the respective joints detected by the position detectors is provided to the image processor 216 via the imaging controller 215 or directly without passing through the imaging controller 215. The optical adapters 224 to 244 mounted on tips of the endoscope arms 22 to 24 include imaging devices 2241 to 2441, and illuminating optical systems 2242 to 2442 for irradiating with lights from the illumination light source 212. The imaging devices 2241 to 2441 are, for example, coupled to the imaging controller 215 via a transmission line (a transmission path: may be wired or wireless). Lenses used for the imaging devices 2241 to 2441 are preferred to be lenses that ensure wide visual fields as much as possible (for example, an angle of view (a viewing angle) is 90 degrees or more and 180 degrees or less), and for example, a foveal lens is employed. For example, a fisheye lens may be employed. The illuminating optical systems 2242 to 2442 are, for example, coupled to the illumination light source 212 via optical fibers. The endoscope arms 22 to 24 may further include the joints on distal end portions so as to change imaging directions of the imaging devices 2241 to 2441 by behavior of the distal end portions. The endoscope arms 22 to 24 have distal ends (for example, at least portions from the last joints 223 to 243 to the tips, and portions inserted into the body (for example, into the abdominal cavity or into the thoracic cavity) of the patient P) that include tubular housings, and the tubular housings are preferred to have small diameters as much as possible. The smaller the diameter is, the more endoscope arms can be inserted into the body of the patient P, while eliminating the need for suturing after surgery even if the endoscope arms are inserted into the body of the patient P. As the tubular housing, for example, a cylinder-shaped housing is applicable. However, not limited to the cylindrical shape, a tubular housing having another shape (for example, may be an elliptical cylindrical shape or a polygonal cylindrical shape) may be employed.

The light source controller 213 responds to the instruction (for example, given via the CPU 211) given from the operator O and the surgical assistant A to adjust an irradiation light amount (strength) and a color of the illumination light source (for example, an LED, a xenon lamp, a mercury lamp, a halogen lamp, a metal halide lamp) 212. Besides the illumination light source, for example, an LD (a laser diode) may be disposed to cause the LD to function as a laser scalpel for opening the organ and the like of the patient or hemostasis with coherent light. In this case, an output of the LD can be controlled by, for example, the light source controller 213.

The imaging controller 215, for example, outputs a control signal via a signal line coupled to the imaging devices 2241 to 2441 to control the imaging devices 2241 to 2441. The imaging controller 215, for example, stores image data output from the imaging devices 2241 to 2441 in an image memory (not illustrated) with a time stamp.

The image processor 216 obtains the images obtained by the respective imaging devices 2241 to 2441 from the image memory (not illustrated). The image processor 216 obtains information (detected by the position detector) on the rotation directions and angles of the plurality of joints 221 to 223, 231 to 233, and 241 to 243, information on the lengths between the respective joints (the movable components), and information on the lengths from the tips of the endoscope arms to the joints closest to the tips, and specifies (calculates) positions of the respective imaging devices 2241 to 2441 in the body (for example, in the abdominal cavity or in the thoracic cavity) of the patient P. The information on the lengths between the respective joints (the movable components), and the information on the lengths from the tips of the endoscope arms to the joints closest to the tips are, for example, preliminarily stored in a memory (not illustrated) (a memory area in the image memory may be used). Based on the information on the specified positions of the imaging devices (for example, information including distances from a reference point, and directions and angles from the reference point) and/or feature points (feature quantities) of the respective images, the image processor 216 joins the images from the three or more imaging devices 2241 to 2441 together to generate a composite image. The feature points of the respective images are, for example, can be extracted using Fourier transform or discrete cosine transform, or performing edge filter processing on the images. After extracting the feature points of the respective images, for example, a method for pattern matching can be used to join the respective images together.

The endoscope arm drive controller 214, for example, receives instructions from the CPU 211 to drive motors (not illustrated) disposed in the respective joints 221 to 223, 231 to 233, and 241 to 243 in response to the instructions so as to cause the endoscope arms 22 to 24 to behave as instructed by the manipulators 12 and 13 of the console apparatus 10, or behave as instructed by the cart-side manipulator 200.

The configuration of the patient-side cart 20 includes, other than the endoscope apparatus 21, for example, the CPU (also referred to as a processor or a controller) 201 that controls behavior of the portions other than the endoscope apparatus 21, a communication apparatus 203 for communicating with the console apparatus 10, the cart-side manipulator 200 with which, for example, the surgical assistant A manipulates the patient-side cart 20, the surgical arm drive controller 204 that drives and controls each of the surgical arms 25 and 26, and at least two surgical arms 25 and 26. While the CPU 211 for the endoscope apparatus 21 is separately disposed from the CPU 201 in Fig. 2, one CPU (any one of the CPU 201 and the CPU 211) may control the entire behavior of the patient-side cart 20 including the endoscope apparatus 21.

The surgical arms 25 and 26 include, for example, the plurality of joints 251 to 254 and 261 to 264, the movable components (no reference numerals) between the respective joints, and surgical instrument adapters 255 and 265 on the distal end portions. The plurality of joints 251 to 254 and 261 to 264 include a plurality of position detectors (not illustrated) associated with the respective joints, thus ensuring detecting an angle and a direction of rotation of each joint. The information on the rotation directions and angles of the respective joints detected by the position detectors is provided to the CPU 201.

The cart-side manipulator 200 may employ various aspects other than a gripping mechanism or a foot pedal without being limited, and is configured with, for example, a joystick, a motion sensor, a switch, or a thumb/finger control.

The communication apparatus 203 receives manipulation instructions from the console apparatus 10, and provides the received instructions to the CPU 201 and the CPU 211 of the endoscope apparatus 21. Based on the received instructions, the CPU 201 and the CPU 211 control the behaviors of the endoscope arms 22 to 24 and the surgical arms 25 and 26 of the endoscope apparatus 21.

The surgical arm drive controller 204 receives the commands from the CPU 201 to drive actuators (not illustrated) disposed in the respective joints 251 to 254 and 261 to 265 in response to the commands so as to cause the surgical arms 25 and 26 to execute the commands instructed by the manipulators 12 and 13 of the console apparatus 10, or execute the commands instructed by the cart-side manipulator 200. The actuators are, for example, servo motors.

### <Composite Image Generation Process>

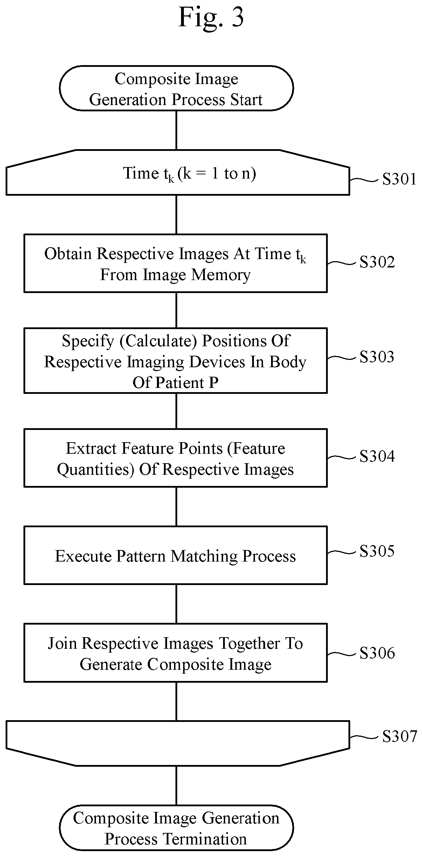

Fig. 3 is a flowchart describing a composite image generation process in the first embodiment. While this composite image generation process is, for example, executed by the image processor 216, when the image processor 216 is implemented by the program as described above, the operation subject is the CPU 211. The following describes the composite image generation process with the image processor 216 as the operation subject, but the description may be understood by replacing the image processor 216 to the CPU 211.

### (i) Step 301 and Step 307

The image processor 216 repeatedly executes processes of Step 302 to Step 306 on each of the images obtained by the imaging devices 2241 to 2441 from a time t₁ to a time tₙ. When the composite image generation process is assumed to be executed from a start of a surgery to a termination of the surgery, for example, a surgery start time is defined as t₁ and a surgery termination time is defined as tₙ. When the imaging devices 2241 to 2441 obtain images of 30 frames per second for example, a time interval for obtaining each image is 1/30 seconds. The composite image generation process may be executed in units of frame, or in units of field (1 field image per 1/60 seconds).

### (ii) Step 302

The image processor 216 reads images (for example, digital images with three or more frames) obtained by the imaging devices 2241 to 2441 at a time tₖ from the image memory (not illustrated). In this Step, the images having three or more frames taken at an identical time are obtained.

### (iii) Step 303

The image processor 216 calculates the positions of the imaging devices 2241 to 2441 respectively mounted on the distal end portions of the endoscope arms 22 to 24, in the body (for example, in the abdominal cavity or in the thoracic cavity) of the patient P. Specifically, the image processor 216 obtains, for example, the information (detected by the position detector) on the rotation directions and angles of the plurality of joints 221 to 223, 231 to 233, 241 to 243, the information on the predetermined lengths between the respective joints (the movable components), and the information on the predetermined lengths from the tips of the endoscope arms to the joints closest to the tips, from the memory (not illustrated). The image processor 216 calculates the distances and the rotation angles of the respective imaging devices from the predetermined reference points based on the information to specify the positions of the respective imaging devices (for example, the position of the joint 231 is set as the reference point, and a space coordinate is formed with this reference point as the origin, thus the distances and the rotation angles from the reference points of the respective imaging devices positioned on the tips of the endoscope arms can be calculated).

### (iv) Step 304

The image processor 216 extracts the feature points of the respective images (The feature point can be referred to as a feature quantity as well. The feature quantity is a feature vector indicating a region around the feature point having great variation of shade with pixel values and differential values). Specifically, the image processor 216, for example, divides the image to be processed into blocks (for example, a block of 8 pixels × 8 pixels), and uses Fourier transform, discrete cosine transform, or similar transform to convert the pixel values of the respective blocks into data in a frequency region. With this process, distributions of frequency components in the respective blocks are obtained, thus ensuring extracting the feature points (the feature quantities) of the respective blocks. Besides Fourier transform and discrete cosine transform, an image in which an edge filter is used to emphasize an edge of each block may be configured as the feature point (the feature quantity).

### (v) Step 305

The image processor 216 executes a pattern matching process on respective images. The pattern matching process may be executed on every region of respective images (for example, correlations are calculated on every pixel and block, and a pixel or a block having the highest correlation value is configured as a pattern matching point), while the pattern matching process may be executed with a limited search range. In this case, speeding up and improving the efficiency of the process are ensured. In the case of limiting the search range, based on the information on the positions of the respective imaging devices 2241 to 2441 specified in Step 303, the range to search the blocks or the pixels where the feature quantities of the respective images match can be determined (for example, configure the regions of 20% of the peripheries of the respective images as the search ranges). The image processor 216 executes the pattern matching process in the search range, and configures matching positions (positions having the highest correlation) in search regions of the respective images as the pattern matching points.

### (vi) Step 306

The image processor 216 joins the respective images together at the positions recognized as the pattern matching points in Step 305 to generate the composite image. The execution of the pattern matching process detects regions where the respective images mutually overlap. The pixel values of the respective images in the overlapping region are slightly different in some cases (for example, since the imaging directions of the respective imaging devices are different, the pixel values obtained by taking images are different in some cases even on an identical object). Then, the images may be joined together after removing the overlapping region from one of the images to be overlapped.

The composite image can be generated with the above-described method. However, the composite image may be generated using a method disclosed in, for example, JP H10-178564 A.

### <Reason for Using Three or More Endoscopes>

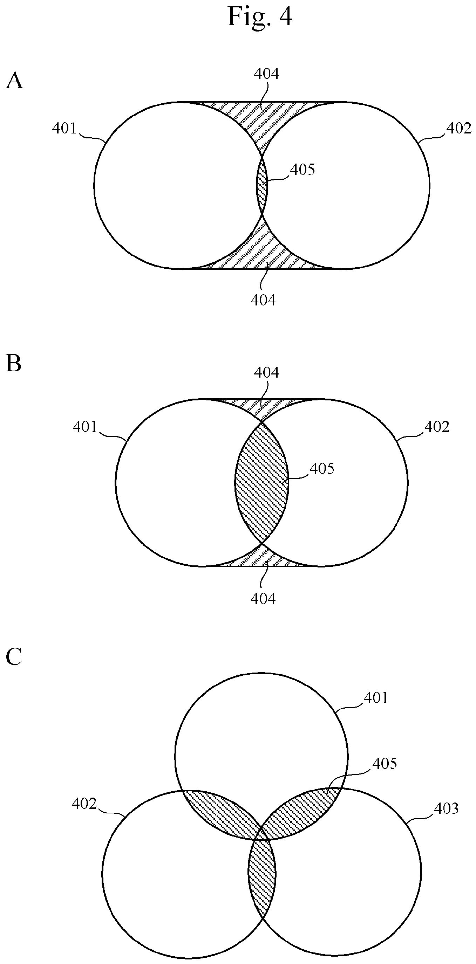

Fig. 4 are drawings schematically illustrating visual fields of the imaging devices to describe a reason why three or more endoscopes are disposed in this disclosure. Fig. 4A and Fig. 4B illustrate visual fields when two imaging devices are disposed, and Fig. 4C illustrates visual fields when three imaging devices are disposed.

For example, when two imaging devices are used to obtain images, a visual field 401 and a visual field 402 of the respective imaging devices need to avoid overlapping as much as possible (in the case of Fig. 4A, an overlapping region 405 is small). In this case, while an area of the overlapping region 405 can be decreased, blind spots 404 occur between the visual field 401 and the visual field 402. Conversely, as illustrated in Fig. 4B, trying to decrease areas of the blind spots 404 decreases a range covered with the visual field 401 and the visual field 402 to increase the area of the overlapping region 405. In view of this, this cannot obtain a visual field sufficient for obtaining the images to observe a state of the surgery and a state of a periphery of the organ as a target of the surgery in the body (for example, in the abdominal cavity or in the thoracic cavity) of the patient.

Therefore, in this disclosure, three or more imaging devices are disposed to use three visual fields, thus attempting to solve the above-described conflicting problem. For example, as illustrated in Fig. 4C, the area of the overlapping region 405 is decreased while eliminating the blind spot 404 formed by overlapping in the case of two visual fields by using the visual field 401, the visual field 402, and a visual field 403 to ensure forming an overall visual field over a wider range.

With the above-described reason, in the first embodiment of this disclosure, the three or more imaging devices 2241 to 2441 are used.

### (2) Second Embodiment

A surgical system according to a second embodiment includes a configuration similar to the surgical system according to the first embodiment. However, in the second embodiment, an endoscope apparatus 21 in a patient-side cart 20 has a different configuration.

The second embodiment discloses a surgical system that includes, for example, a patient-side cart (also referred to as a surgical robot) that includes an endoscope apparatus including an endoscope arm configured to have a function to rotate at least a distal end portion of a tubular housing of an endoscope, and a console apparatus for manipulating the patient-side cart. In the endoscope apparatus, for example, an imaging device has an optical axis having a predetermined angle with a rotation axis of the distal end portion of the tubular housing, images obtained by the imaging device while rotating the distal end portion of the tubular housing of the endoscope are joined together to generate a composite image, and the composite image is indicated on a display screen. Thus rotating the distal end portion of the endoscope provides the operator with the image of a wide visual field having a position of the endoscope as the center, and the operator can perform a surgery while confirming a state of an affected part and a desired position around the affected part by a visual check (ensuring an endoscopic surgery while having a large visual field as in a laparotomy). In this case, as a lens of the imaging device, for example, a foveal lens can be used as well.

### <Configuration of Patient-Side Cart 20>

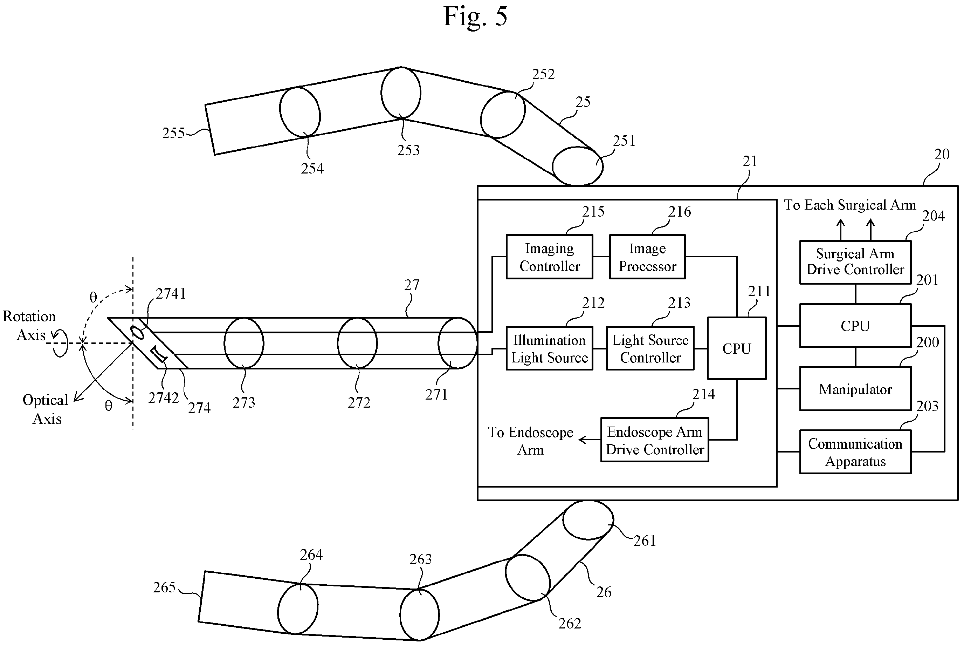

Fig. 5 is a drawing illustrating an exemplary configuration of the patient-side cart 20 according to the second embodiment. A point different from the first embodiment is that one endoscope arm 27 is mounted and the endoscope arm 27 has a distal end portion on which an optical adapter 274 (including an imaging device 2741 and an illuminating optical system 2742) is configured to be rotatable around a rotation axis. The endoscope arm 27 has an obliquely formed distal end surface, and an optical axis of the imaging device 2741 has a predetermined angle with the rotation axis. The angle between the optical axis of the imaging device 2741 and the rotation axis can be configured to be, for example, 1/2 of an angle of view (a viewing angle) of a lens of the imaging device 2741. In this case, the distal end surface of the endoscope arm 27 may have an inclined angle of, for example, 1/2 of the angle of view of the lens of the imaging device 2741 as well.

In the second embodiment, the imaging device 2741 obtains images during surgery while the optical adapter 274 on the distal end portion of the endoscope arm 27 is rotated around the rotation axis. The rotation of the optical adapter 274 on the distal end portion of the endoscope arm 27 is controlled by an endoscope arm drive controller 214. Specifically, a joint 273 includes a small (low speed) actuator for rotating the distal end portion separately from an actuator for driving the joint, and the distal end portion of the endoscope arm 27 can be rotated by a rotation drive mechanism (including a rotation drive shaft coupled to the distal end portion and various gears for transmitting rotation drive (for example, a gear such as a bevel gear couples the distal end portion to a shaft of the small motor in some cases)). For example, the endoscope arm drive controller 214 is configured to control a rotation speed and similar factor of the distal end portion. The rotation of the distal end portion of the endoscope arm 27 can be detected by a position detector (for example, an encoder). For example, a position detector disposed on a joint closest to the distal end portion can be used.

The imaging controller 215 stores images obtained by the imaging device 2741 while rotating in an image memory (not illustrated) with time stamps.

An image processor 216 generates a composite image while performing matching among the images obtained by the imaging controller 215. A composite image generation process will be described later.

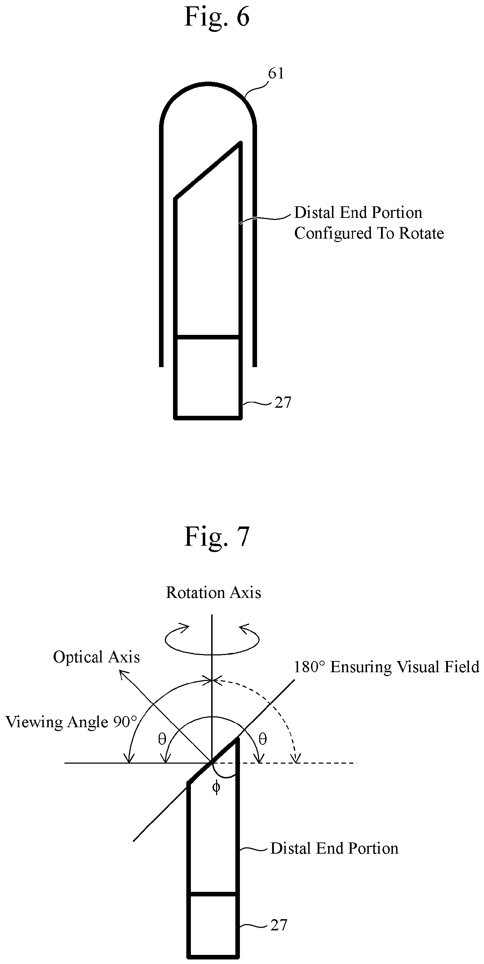

As illustrated in Fig. 6, the distal end portion of the endoscope arm 27 is configured to be covered with a transparent sheath 61 having a rounded distal end portion. This prevents body tissue of the patient P from being damaged during surgery.

### <Visual Field Ensured in Rotating Distal End Portion>

Fig. 7 is a drawing describing a visual field ensured in rotating the distal end portion of the endoscope arm 27.

In Fig. 7, a viewing angle of the lens of the imaging device is configured to be, for example, 90 degrees. The angle between the optical axis of the lens and the rotation axis is configured to be, for example, 45 degrees. In this configuration, rotating the distal end portion of the endoscope arm 27 around the rotation axis ensures a viewing angle θ of 180 degrees as double of 90 degrees by the rotation, thus ensuring the visual field in a wider range. For example, assuming that the viewing angle of the lens is 120 degrees, the angle between the optical axis and the rotation axis is preferred to be 60 degrees (an inclined angle ϕ of the distal end surface is 30 degrees). In this case, the viewing angle θ ensured by the rotation is 240 degrees as double of 120 degrees. For example, when a lens having the viewing angle of 180 degrees is disposed on the distal end portion of the endoscope arm 27 such that the angle between the optical axis and the rotation axis is 90 degrees, the viewing angle θ of 360 degrees is theoretically ensured.

### <Composite Image Generation Process>

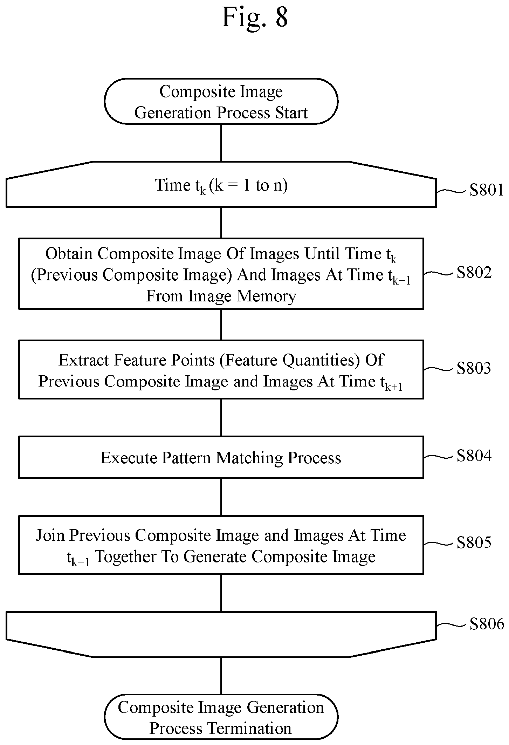

Fig. 8 is a flowchart describing a composite image generation process in the second embodiment. While this composite image generation process is, for example, executed by the image processor 216, when the image processor 216 is implemented by the program as described above, the operation subject is a CPU 211. The following describes the composite image generation process with the image processor 216 as the operation subject, but the description may be understood by replacing the image processor 216 to the CPU 211.

### (i) Step 801 and Step 806

The image processor 216 repeatedly executes processes of Step 802 to Step 805 on each of the images obtained by the imaging device 2741 from a time t₁ to a time tₙ. When the composite image generation process is assumed to be executed from a start of a surgery to a termination of the surgery, for example, a surgery start time is defined as t₁ and a surgery termination time is defined as tn. When the imaging device 2741 obtains images of 30 frames per second for example, a time interval for obtaining each image is 1/30 seconds. The composite image generation process may be executed in units of frame, or in units of field (1 field image per 1/60 seconds).

### (ii) Step 802

The image processor 216 reads a composite image (a composite image generated in a previous process: a previous composite image) generated by using the images until a time tₖ and images obtained by the imaging device 2741 from an image memory (not illustrated). In a case (a case of t₁) where the composite image has not yet been generated at an early stage of the process start, the images between the time t₁ and the time t₂ are read from the image memory instead of the composite image.

### (iii) Step 803

The image processor 216 extracts feature points (feature quantities) of the previous composite image and the image at a time tₖ₊₁. Specifically, the image processor 216, for example, divides the image to be processed into blocks (for example, a block of 8 pixels × 8 pixels), and uses Fourier transform, discrete cosine transform, or similar transform to convert the pixel values of the respective blocks into data in a frequency region. With this process, distributions of frequency components in the respective blocks are obtained, thus ensuring extracting the feature quantities of the respective blocks. Besides Fourier transform and discrete cosine transform, an image in which an edge filter is used to emphasize an edge of each block may be configured as the feature quantity. When the feature quantity has been already extracted from the previous composite image, for example, it is only necessary to read from the image memory (not illustrated).

### (iv) Step 804

The image processor 216 executes a pattern matching process on the previous composite image and the image at the time tₖ₊₁. In the second embodiment, the rotating imaging device 2741 ensures the execution of the pattern matching equivalent to the pattern matching on a moving image. Specifically, the image processor 216, for example, preliminarily executes the pattern matching process in a search range (the rotation speed can be used to calculate a moving distance of the image (a shifted range of the image) in one frame period (for example, 1/60 seconds), and the search range can be configured with the range and a margin considering an error), and configures matching positions (positions having the highest correlation) in search regions of the respective images as the pattern matching points.

### (v) Step 805

The image processor 216 joins the previous composite image and the image at the time tₖ₊₁ together at the position recognized as the pattern matching point in Step 804 to generate a new composite image. One rotation of the distal end portion of the endoscope arm 27 generates a composite image of the viewing angle θ. The images obtained by the second rotation or later are sequentially superimposed on an appropriate position (a position where the matching is confirmed) of the already generated composite image.

The composite image can be generated with the above-described method. However, the composite image may be generated using a method disclosed in, for example, JP H10-178564 A.

### <Low Resolution Mode and High Resolution Mode>

In this embodiment, for example, the images obtained while rotating the distal end portion of the endoscope arm 27 are provided in low resolution (low image quality: in other words, a first resolution or a first image quality), and therefore, the composite image obtained by composing those images is also indicated on the display screen of the display apparatus 30 with low resolution. On the other hand, the images obtained while stopping the rotation of the distal end portion are indicated on the display screen of the display apparatus 30 with high resolution (high image quality: in other words, a second resolution or a second image quality (higher resolution compared with the first resolution)).

For example, when the operator O finds an anxious part and the like on the composite image and wants to intensively observe the part, the operator O can manipulate the console apparatus 10 to instruct to stop the rotation of the distal end portion, and instruct the part desired to be further intensively observed to confirm the part on the high resolution image.

The endoscope apparatus and the surgical system according to the embodiment can decrease the number of the endoscope inserted into the body of the patient P compared with the first embodiment, thus making a surgery more minimally invasive.

### (3) Third Embodiment

A surgical system according to a third embodiment includes a configuration similar to the surgical system according to the first embodiment. However, in the third embodiment, an endoscope apparatus 21 in a patient-side cart 20 has a different configuration. In the second embodiment, the endoscope apparatus 21 includes only one endoscope arm 27 having a distal end portion configured to rotate, while the third embodiment is different in a point that three or more endoscope arms having distal end portions configured to rotate are disposed.

The third embodiment discloses a surgical system that includes, for example, a patient-side cart (also referred to as a surgical robot) and a console apparatus for manipulating the patient-side cart. The patient-side cart includes an endoscope apparatus having three or more endoscopes and three or more endoscope arms, and the three or more endoscope arms have a function to rotate at least a distal end portion of a tubular housing of each endoscope. In the endoscope apparatus, for example, imaging devices each have an optical axis having a predetermined angle with a rotation axis of the distal end portion of each tubular housing, three or more images obtained by the imaging devices while rotating the distal end portions of the tubular housings of the respective endoscopes are joined together to generate a composite image, and the composite image is indicated on a display screen. Thus rotating the distal end portions of the three or more endoscopes provides the operator with the image in the visual field of further wide range (for example, 360 degrees), and the operator can perform a surgery while confirming a state of an affected part and a desired position around the affected part in a wide range by a visual check (ensuring an endoscopic surgery while having a large visual field as in a laparotomy). In this case, as a lens of the imaging device, for example, a foveal lens can be used as well.

For example, when the operator instructs it to stop the rotation of the distal end portion of each endoscope arm, the images in the states where the rotations are stopped are indicated on the display screen. In this case, for example, the images obtained and indicated in the stopped state can be provided in high image qualities compared with the images obtained while rotating the distal end portions. Accordingly, the operator can confirm the desired portion in more detail by a visual check. In this case, for example, the operator can give an instruction of information on a position of a target in the body of which the image is to be obtained in the state where the rotation is stopped. At this time, for example, the image obtained by only the imaging device with which the image of the target position is obtainable is indicated on the display screen.

### <Configuration of Patient-Side Cart>

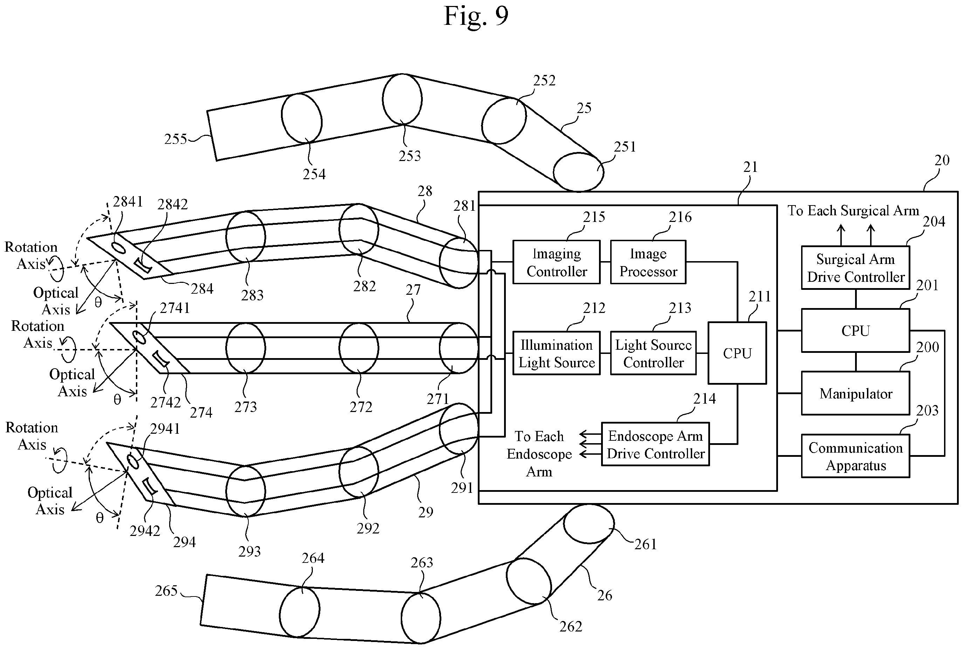

Fig. 9 is a drawing illustrating an exemplary configuration of the patient-side cart 20 according to the third embodiment. A point different from the first embodiment is that three or more endoscope arms 27 to 29 have distal end portions configured to rotate similarly to the second embodiment. A point different from the second embodiment is that the three or more endoscope arms 27 to 29 having the distal end portions configured to rotate are disposed.

Similarly to the second embodiment, the endoscope arms 27 to 29 have obliquely formed distal end surfaces, and optical axes of imaging devices 2741 to 2941 each have a predetermined angle with each of the rotation axes of the distal end portions. The angles between the optical axes of the imaging devices 2741 to 2941 and the rotation axes can be each configured to be, for example, 1/2 of an angle of view (a viewing angle) of each lens of the imaging devices 2741 to 2941. In this case, the distal end surfaces of the endoscope arms 27 to 29 may have an inclined angle of, for example, (90 - the viewing angle of the lens of the imaging device 2741 × 1/2) degrees.

In the third embodiment, for example, the imaging devices 2741 to 2941 obtain images during surgery while optical adapters 274 to 294 on the distal end portions of the endoscope arms 27 to 29 are each rotated around the rotation axis. The rotations of the optical adapters 274 to 294 on the distal end portions of the endoscope arms 27 to 29 are controlled by, for example, an endoscope arm drive controller 214. Specifically, for example, joints 273 to 293 each include a small (low speed) motor for rotating the distal end portion separately from a motor for driving the joint, and the distal end portions of the endoscope arms 27 to 29 can be rotated by a rotation drive mechanism (including a rotation drive shaft coupled to the distal end portion and various gears for transmitting rotation drive (for example, a gear such as a bevel gear couples the distal end portion to a shaft of the small motor in some cases)). For example, the endoscope arm drive controller 214 is configured to control a rotation speed and similar factor of the distal end portion. The rotation of each distal end portion of the endoscope arms 27 to 29 can be detected by a position detector (for example, an encoder). For example, respective position detectors disposed on joints closest to the respective distal end portions can be used.

The imaging controller 215 stores images obtained by the imaging devices 2741 to 2941 while rotating in an image memory (not illustrated) with time stamps.

An image processor 216 generates a composite image while performing matching among the images obtained by the imaging controller 215. A composite image generation process will be described later.

Similarly to the second embodiment, as illustrated in Fig. 6, the distal end portions of the endoscope arms 27 to 29 are configured to be covered with, for example, transparent sheaths 61 having rounded distal end portions. This prevents body tissue of the patient P from being damaged during surgery.

### <Composite Image Generation Process>

The composite image generation process in the third embodiment can be realized by combining the composite image generation process (Fig. 3) in the first embodiment with the composite image generation process (Fig. 8) in the second embodiment. For example, the composite image of the images obtained by taking while rotating each of the imaging devices 2741 to 2941 of the three or more endoscope arms 27 to 29 is generated in accordance with the process in Fig. 8. This generates three or more rotation composite images (also referred to as around view images). The three or more rotation composite images are further combined in accordance with the process in Fig. 3, thus generating a final composite image.

### <Low Resolution Mode and High Resolution Mode>

In this embodiment, similarly to the second embodiment, for example, the images obtained while rotating the distal end portions of the endoscope arms 27 to 29 are provided in low resolution (low image quality: in other words, a first resolution or a first image quality), and therefore, the composite image of the rotation images of the respective distal end portions and the final composite image obtained by further joining the three or more composite images are also indicated on the display screen of the display apparatus 30 with low resolution. On the other hand, similarly to the second embodiment, the images obtained while stopping the rotation of the distal end portions are indicated on the display screen of the display apparatus 30 with high resolution (high image quality: in other words, a second resolution or a second image quality (higher resolution compared with the first resolution)).

For example, when the operator O finds an anxious part and the like on the final composite image and wants to intensively observe the part, the operator O can manipulate the console apparatus 10 to instruct to stop the rotation of at least one distal end portion covering the part to be intensively observed among the distal end portions of the three or more endoscope arms 27 to 29, and instruct the part desired to be further intensively observed to confirm the part on the high resolution image.

### (4) Modification

(i) While, in the first to the third embodiments, the composite image indicating a current state in the body (for example, in the abdominal cavity or in the thoracic cavity) of the patient during surgery is displayed on the display screen of the display apparatus 30, past images (irrespective of the composite image or not) may be displayed on the display screen together (for example, on a multi-screen). For example, a group of the images from a time point where the distal end portions (the portions on which the imaging devices are mounted) of the endoscope arms 22 to 24 and the distal end portions of the endoscope arms 27 to 29 are inserted into the body of the patient P to a time point where those distal end portions move to a position for taking images of the surgical site are stored in the image memory (not illustrated). Therefore, displaying the images near insertion routes of the respective endoscope arms and the images on peripheries of insertion routes of surgical instruments mounted on the respective surgical arms (both are images taken in the past) with the current images during surgery on the display screen ensures, for example, the operator O to confirm whether the surgical instruments manipulated by the operator O himself/herself are inserted excessively pressing the organs in the body of the patient P.
   The functions and the configurations in each embodiment may be appropriately combined for use. For example, when the endoscope apparatus includes the three or more endoscope arms 22 to 24, it is not necessary to configure every distal end portion of the three or more endoscope arms to be rotatable (a configuration where the distal end portion of at least one endoscope arm is rotatable may be employed).
(ii) The functions of each embodiment can be realized by program codes of software. In this case, a storage medium in which the program codes are recorded is provided to a system or an apparatus, and a computer (or a CPU and/or an MPU) in the system or the apparatus reads the program codes stored in the storage medium. In this case, the program code itself read from the storage medium realizes the above-described function of the embodiment, and the program code itself and the storage medium that stores the program code are included in this embodiment. As the storage medium for supplying such program codes, for example, a flexible disk, a CD-ROM, a DVD-ROM, a hard disk, an optical disk, a magneto-optical disk, a CD-R, a magnetic tape, a nonvolatile memory card, and a ROM are used.

Based on the instruction of the program code, an OS (operating system) operating on a computer or similar system performs a part of or all the actual process, and with the process, the above-described function of the embodiment may be achieved. Furthermore, the program code read from the storage medium is written on a memory on the computer, subsequently, based on the instruction of this program code, a CPU or the like of the computer performs a part of or all the actual process, and with the process, the above-described function of the embodiment may be achieved.

Furthermore, the program code of the software achieving the function of the embodiment is delivered via a network, the program code is stored in the storage means such as the hard disk and the memory or the storage medium such as the CD-RW and the CD-R in the system or the apparatus, and the computer (or the CPU and/or the MPU) in the system or the apparatus may read and execute the program code stored in the storage means or the storage medium when used.

### Reference Signs List

- 1: Surgical system
- 10: Console apparatus
- 20: Patient-side cart
- 21: Endoscope apparatus
- 22, 23, 24, 27, 28, 29: Endoscope arm
- 25, 26: Surgical arm
- 30: Display apparatus

## Claims

1. An endoscope apparatus (21) comprising:
one or more endoscopes each of which includes an imaging device (2741, 2841, 2941) on a distal end portion of a tubular housing, the one or more endoscopes configured to obtain an image in a body of a subject, the imaging device (2741, 2841, 2941) having an optical axis having a predetermined angle with a rotation axis of the distal end portion of the tubular housing, the rotation axis extending in a direction identical to a longitudinal direction of the tubular housing and the imaging device being inclined with respect to the rotation axis;
one or more endoscope arms (27, 28, 29) each of which includes a plurality of joints (272 - 273, 282 - 283, 292 - 293) and a plurality of position detectors corresponding to respective plural joints, and is configured to hold the respective endoscope, and
a controller (211, 215, 215) configured to process a plurality of images obtained by the one or more endoscopes, the plurality of images are obtained by the imaging device by rotating the distal end portion of the tubular housing of the endoscope,
a rotation driver (214) configured to rotate at least the distal end portion of the tubular housing of the one or more endoscopes,
wherein the controller (211, 215, 215) uses position information of the imaging device and feature points of the respective images obtained by the imaging device (2741, 2841, 2941) of the one or more endoscope arms (27, 28, 29) to join the respective images together, to generate a composite image, the position information being obtained by the position detectors.

2. The endoscope apparatus according to claim 1, comprising:
three or more of the endoscopes; wherein the controller is electrically coupled to the three or more endoscopes, the controller configured to process the images obtained by the respective three or more endoscopes.
wherein the plurality of images are obtained by the imaging devices of the three or more endoscopes.

3. The endoscope apparatus according to claim 2,
wherein at least three images obtained by the three or more endoscopes have mutually overlapping regions, and
the controller uses the feature points of the respective images to specify the mutually overlapping regions, and generates the composite image.

4. The endoscope apparatus according to claim 2 or 3,
wherein the imaging devices included in the three or more endoscopes each include a lens having a viewing angle equal to or more than 90 degrees.

5. The endoscope apparatus according to any one of claims 2 to 4, further comprising,
a memory to store images,
wherein the controller stores images before composition obtained by the respective imaging devices since the three or more endoscopes were inserted into the body of the subject and the composite images since the three or more endoscopes were inserted into the body of the subject in the memory, and the controller reads the composite images at a predetermined time point from the memory in response to an input instruction.

6. The endoscope apparatus according to claim 1,
wherein the predetermined angle between the optical axis of the imaging device (2741, 2841, 2941) and the rotation axis is 1/2 of a viewing angle of a lens of the imaging device (2741, 2841, 2941).

7. The endoscope apparatus according to any one of claims 1 to 6,
wherein the lens of the imaging device (2741, 2841, 2941) is a foveal lens.

8. The endoscope apparatus according to any one of claims 1 to 7,
wherein a sheath (61) is mounted on the distal end portion of the tubular housing.

9. The endoscope apparatus according to any one of claims 1 to 8,
wherein the controller (211, 215, 215) stops the rotation of the distal end portions of the respective tubular housings in response to an input instruction, and obtains the plurality of images by at least one of the imaging devices (2741, 2841, 2941) in a state where the rotation is stopped.

10. The endoscope apparatus according to claim 9,
wherein the input instruction includes information on position of a target in the body, an image of the target being obtained in the state where the rotation is stopped, and
the controller (211, 215, 215) obtains an image obtained by only the imaging device with which the image of the target position is obtainable.

11. An endoscope system, comprising:
a robot cart (20) that includes an endoscope apparatus according to any one of claims 1-10
a console apparatus (10) that transmits an instruction to manipulate the three or more endoscope arms; and
a display apparatus (30) that displays a composite image generated by the endoscope apparatus on a display screen.

## Patentansprüche

1. Eine Endoskopvorrichtung (21), umfassend:
ein oder mehrere Endoskope, von denen jedes eine Abbildungsvorrichtung (2741, 2841, 2941) an einem distalen Endabschnitt eines röhrenförmigen Gehäuses aufweist, wobei das eine oder die mehreren Endoskope konfiguriert sind, ein Bild in einem Körper eines Subjekts zu erhalten, wobei die Abbildungsvorrichtung (2741, 2841, 2941) eine optische Achse aufweist, die einen vorbestimmten Winkel mit einer Rotationsachse des distalen Endabschnitts des röhrenförmigen Gehäuses aufweist, wobei sich die Rotationsachse in einer Richtung erstreckt, die identisch mit einer Längsrichtung des röhrenförmigen Gehäuses ist, und die Abbildungsvorrichtung in Bezug auf die Rotationsachse geneigt ist;
einen oder mehrere Endoskoparme (27, 28, 29), von denen jeder eine Mehrzahl von Gelenken (272 - 273, 282 - 283, 292 - 293) und eine Mehrzahl von Positionsdetektoren, die den jeweiligen mehreren Gelenken entsprechen, aufweist und konfiguriert ist, das jeweilige Endoskop zu halten, und
eine Steuerung (211, 215, 215), die konfiguriert ist, eine Vielzahl von Bildern zu verarbeiten, die durch das eine oder die mehreren Endoskope erhalten werden, wobei die Vielzahl von Bildern durch die Bildgebungsvorrichtung durch Drehen des distalen Endabschnitts des röhrenförmigen Gehäuses des Endoskops erhalten wird,
einen Rotationstreiber (214), der konfiguriert ist, zumindest den distalen Endabschnitt des rohrförmigen Gehäuses des einen oder der mehreren Endoskope zu drehen,
wobei die Steuerung (211, 215, 215) Positionsinformationen der Abbildungsvorrichtung und Merkmalspunkte der jeweiligen Bilder, die durch die Abbildungsvorrichtung (2741, 2841, 2941) des einen oder der mehreren Endoskoparme (27, 28, 29) erhalten werden, verwendet, um die jeweiligen Bilder zusammenzufügen, um ein zusammengesetztes Bild zu erzeugen, wobei die Positionsinformationen durch die Positionsdetektoren erhalten werden.

2. Die Endoskopvorrichtung nach Anspruch 1, umfassend:
drei oder mehr der Endoskope; wobei
die Steuerung elektrisch mit den drei oder mehr Endoskopen gekoppelt ist, wobei die Steuerung konfiguriert ist, die von den jeweiligen drei oder mehr Endoskopen erhaltenen Bilder zu verarbeiten,
wobei die Vielzahl von Bildern durch die Bildgebungsvorrichtungen der drei oder mehr Endoskope erhalten werden.

3. Die Endoskopvorrichtung nach Anspruch 2,
wobei mindestens drei durch die drei oder mehr Endoskope erhaltene Bilder sich gegenseitig überlappende Bereiche aufweisen, und
die Steuerung die Merkmalspunkte der jeweiligen Bilder verwendet, um die sich gegenseitig überlappenden Bereiche zu spezifizieren, und das zusammengesetzte Bild erzeugt.

4. Die Endoskopvorrichtung nach Anspruch 2 oder 3,
wobei die Abbildungsvorrichtungen, die in den drei oder mehr Endoskopen enthalten sind, jeweils eine Linse mit einem Betrachtungswinkel von gleich oder mehr als 90 Grad aufweisen.

5. Die Endoskopvorrichtung nach einem der Ansprüche 2 bis 4, ferner umfassend
einen Speicher zum Speichern von Bildern,
wobei die Steuerung Bilder vor der Zusammensetzung, die von den jeweiligen Bildgebungsvorrichtungen erhalten wurden, seit die drei oder mehr Endoskope in den Körper des Subjekts eingeführt wurden, und die zusammengesetzten Bilder, seit die drei oder mehr Endoskope in den Körper des Subjekts eingeführt wurden, in dem Speicher speichert, und die Steuerung die zusammengesetzten Bilder zu einem vorbestimmten Zeitpunkt als Reaktion auf einen Eingabebefehl aus dem Speicher liest.

6. Die Endoskopvorrichtung nach Anspruch 1,
wobei der vorbestimmte Winkel zwischen der optischen Achse der Abbildungsvorrichtung (2741, 2841, 2941) und der Drehachse 1/2 eines Betrachtungswinkels einer Linse der Abbildungsvorrichtung (2741, 2841, 2941) beträgt.

7. Die Endoskopvorrichtung nach einem der Ansprüche 1 bis 6,
wobei das Objektiv der Abbildungsvorrichtung (2741, 2841, 2941) ein foveales Objektiv ist.

8. Die Endoskopvorrichtung nach einem der Ansprüche 1 bis 7,
wobei an dem distalen Endabschnitt des rohrförmigen Gehäuses eine Hülse (61) angebracht ist.

9. Die Endoskopvorrichtung nach einem der Ansprüche 1 bis 8,
wobei die Steuerung (211, 215, 215) die Drehung der distalen Endabschnitte der jeweiligen rohrförmigen Gehäuse in Reaktion auf eine Eingabeanweisung anhält und die mehreren Bilder durch mindestens eine der Abbildungsvorrichtungen (2741, 2841, 2941) in einem Zustand erhält, in dem die Drehung angehalten ist.

10. Die Endoskopvorrichtung nach Anspruch 9,
wobei die Eingabeanweisung Informationen über die Position eines Ziels im Körper enthält, wobei ein Bild des Ziels in dem Zustand erhalten wird, in dem die Drehung gestoppt ist, und
die Steuerung (211, 215, 215) ein Bild erhält, das nur von der Bildgebungsvorrichtung erhalten wird, mit der das Bild der Zielposition erhalten werden kann.

11. Ein Endoskopsystem, umfassend
einen Roboterwagen (20), der eine Endoskopvorrichtung nach einem der Ansprüche 1 bis 10 enthält
eine Konsolenvorrichtung (10), die eine Anweisung zum Manipulieren der drei oder mehr Endoskoparme überträgt; und
eine Anzeigevorrichtung (30), die ein von der Endoskopvorrichtung erzeugtes zusammengesetztes Bild auf einem Anzeigebildschirm anzeigt.

## Revendications

1. Appareil d'endoscope (21) comprenant :
un ou plusieurs endoscopes dont chacun inclut un dispositif d'imagerie (2741, 2841, 2941) sur une partie d'extrémité distale d'un boîtier tubulaire, le ou les endoscopes étant configurés pour obtenir une image dans un corps d'un sujet, le dispositif d'imagerie (2741, 2841, 2941) présentant un axe optique présentant un angle prédéterminé avec un axe de rotation de la partie d'extrémité distale du boîtier tubulaire, l'axe de rotation s'étendant dans une direction identique à une direction longitudinale du boîtier tubulaire et le dispositif d'imagerie étant incliné par rapport à l'axe de rotation ;
un ou plusieurs bras d'endoscope (27, 28, 29) dont chacun inclut une pluralité d'articulations (272 - 273, 282 - 283, 292 - 293) et une pluralité de détecteurs de positions correspondant à plusieurs articulations respectives, et sont configurés pour maintenir l'endoscope respectif, et
un dispositif de commande (211, 215, 215) configuré pour traiter une pluralité d'images obtenues par le ou les endoscopes, la pluralité d'images sont obtenues par le dispositif d'imagerie par rotation de la partie d'extrémité distale du boîtier tubulaire de l'endoscope,
un dispositif d'entraînement en rotation (214) configuré pour faire tourner au moins la partie d'extrémité distale du boîtier tubulaire du ou des endoscopes,
dans lequel le dispositif de commande (211, 215, 215) utilise des informations de position du dispositif d'imagerie et des points caractéristiques des images respectives obtenues par le dispositif d'imagerie (2741, 2841, 2941) du ou des bras d'endoscope (27, 28, 29) pour réunir les images respectives, pour générer une image composite, les informations de position étant obtenues par les détecteurs de position.

2. Appareil d'endoscope selon la revendication 1, comprenant :
trois, ou plus, des endoscopes ; dans lequel
le dispositif de commande est couplé électriquement aux trois, ou plus, endoscopes, le dispositif de commande étant configuré pour traiter les images obtenues par les trois, ou plus, endoscopes respectifs,
dans lequel la pluralité d'images sont obtenues par les dispositifs d'imagerie des trois, ou plus, endoscopes.

3. Appareil d'endoscope selon la revendication 2,
dans lequel au moins trois images obtenues par les trois, ou plus, endoscopes présentent des régions se chevauchant mutuellement et
le dispositif de commande utilise les points caractéristiques des images respectives pour spécifier les régions se chevauchant mutuellement et génère l'image composite.

4. Appareil d'endoscope selon la revendication 2 ou 3,
dans lequel les dispositifs d'imagerie inclus dans les trois, ou plus, endoscopes incluent chacun une lentille présentant un angle de visualisation égal ou supérieur à 90 degrés.

5. Appareil d'endoscope selon l'une quelconque des revendications 2 à 4, comprenant en outre
une mémoire pour stocker des images,
dans lequel le dispositif de commande stocke des images avant une composition obtenue par les dispositifs d'imagerie respectifs puisque les trois, ou plus, endoscopes ont été insérés dans le corps du sujet et les images composites puisque les trois, ou plus, endoscopes ont été insérés dans le corps du sujet dans la mémoire, et le dispositif de commande lit les images composites à un moment prédéterminé à partir de la mémoire en réponse à une instruction d'entrée.

6. Appareil d'endoscope selon la revendication 1,
dans lequel l'angle prédéterminé entre l'axe optique du dispositif d'imagerie (2741, 2841, 2941) et l'axe de rotation est 1/2 d'un angle de visualisation d'une lentille du dispositif d'imagerie (2741, 2841, 2941).

7. Appareil d'endoscope selon l'une quelconque des revendications 1 à 6,
dans lequel la lentille du dispositif d'imagerie (2741, 2841, 2941) est une lentille fovéale.

8. Appareil d'endoscope selon l'une quelconque des revendications 1 à 7,
dans lequel une gaine (61) est montée sur la partie d'extrémité distale du boîtier tubulaire.

9. Appareil d'endoscope selon l'une quelconque des revendications 1 à 8,
dans lequel le dispositif de commande (211, 215, 215) arrête la rotation des parties d'extrémité distale des boîtiers tubulaires en réponse à une instruction d'entrée et obtient la pluralité d'images par au moins un des dispositifs d'imagerie (2741, 2841, 2941) dans un état où la rotation est arrêtée.

10. Appareil d'endoscope selon la revendication 9,
dans lequel l'instruction d'entrée inclut des informations concernant la position d'une cible dans le corps, une image de la cible étant obtenue dans l'état où la rotation est arrêtée, et
le dispositif de commande (211, 215, 215) obtient une image obtenue par le seul dispositif d'imagerie avec lequel l'image de la position cible peut être obtenue.

11. Système d'endoscope comprenant :
un chariot de robot (20) qui inclut un appareil d'endoscope selon l'une quelconque des revendications 1 - 10,
un appareil de console (10) qui transmet une instruction pour manipuler les trois, ou plus, bras d'endoscope ; et
un appareil d'affichage (30) qui affiche une image composite générée par l'appareil d'endoscope sur un écran d'affichage.
